(19) Europäisches Patentamt
European Patent Office
Office européen des brevets

(11) **EP 3 378 466 A1**

(12) **EUROPEAN PATENT APPLICATION**
published in accordance with Art. 153(4) EPC

(43) Date of publication:
**26.09.2018 Bulletin 2018/39**

(21) Application number: **16865931.6**

(22) Date of filing: **15.11.2016**

(51) Int Cl.:
*A61K 8/55* (2006.01)    *A61K 8/24* (2006.01)
*A61Q 11/00* (2006.01)

(86) International application number:
**PCT/JP2016/004885**

(87) International publication number:
**WO 2017/085927 (26.05.2017 Gazette 2017/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **18.11.2015 JP 2015225560**

(71) Applicant: **Kabushiki Kaisha Sangi**
**Tokyo 104-8440 (JP)**

(72) Inventors:
• **KASUGA, Ayako**
  **Tokyo 104-8440 (JP)**
• **OBUKI, Mariko**
  **Tokyo 104-8440 (JP)**
• **TAKAMATSU, Rie**
  **Tokyo 104-8440 (JP)**
• **TAKIKAWA, Rimiko**
  **Tokyo 104-8440 (JP)**

(74) Representative: **McGuire, Gillian Margaret**
**Carpmaels & Ransford LLP**
**One Southampton Row**
**London WC1B 5HA (GB)**

(54) **COMPOSITION FOR USE IN ORAL CAVITY**

(57) It is an object to provide an oral composition having a high remineralizing effect on demineralized tooth enamel. The present invention is an oral composition comprising lecithin and a calcium phosphate, wherein a content of the calcium phosphate is 0.0001 to 30% by mass. The present invention is a tooth remineralizing agent comprising lecithin and a calcium phosphate as active ingredients.

EP 3 378 466 A1

## Description

## Technical Field

The present invention relates to an oral composition having a tooth remineralizing effect.

## Background Art

Caries begins with the adhesion of tooth decay bacteria such as *streptococcus mutans* bacteria to a tooth surface and the formation of plaque, and an organic acid produced by the metabolism of foodstuff by the tooth decay bacteria in the plaque demineralizes tooth enamel to cause a state of initial caries. Saliva has the function of remineralizing this demineralized portion by the function of calcium and phosphorus in the saliva and returning the tooth to the original state. If the demineralized tooth can be sufficiently regenerated by remineralization, the occurrence of caries can be suppressed.

Accordingly, dentifrices in which a fluoride and hydroxyapatite that is one of calcium phosphates and has a crystal structure similar to that of the inorganic component of teeth are blended for accelerating the remineralization of teeth are produced and sold.

However, the remineralization of demineralized portions is not sufficient only with saliva and the use of a dentifrice in which a fluoride or hydroxyapatite is blended, and the development of an oral composition, such as a dentifrice, that can sufficiently achieve remineralization is required.

Accordingly, an oral dentifrice in which hydroxyapatite and tricalcium phosphate having a particle diameter of 0.05 $\mu$m to 1.0 $\mu$m are blended and which can restore and protect minute irregularities on tooth surfaces, prevent tooth decay, strengthen dentin, and enhance a whitening effect (Patent Document 1), an oral composition in which a hydroxyapatite fine powder is blended in a water-soluble cellulose solution, thereby being able to allow the hydroxyapatite fine powder to remain long on tooth surfaces (Patent Document 2), an oral composition in which a sugar alcohol such as xylitol and calcium secondary phosphate are used in combination, thereby being able to significantly accelerate remineralization (Patent Document 3), an oral composition in which low crystalline hydroxyapatite is blended and which can prevent diseases and an uncomfortable feeling in the oral cavity by adsorbing to bacteria in the oral cavity for disinfection (Patent Document 4), an oral composition in which a calcium compound such as hydroxyapatite is blended in royal jelly or an extract thereof and which can whiten teeth, prevent tooth decay by remineralization, and prevent periodontal diseases (Patent Document 5), a dentifrice composition in which a calcium compound such as hydroxyapatite is blended in an ultramarine blue composition and which is caused by a tooth remineralizing (Patent Document 6), a remineralization accelerating agent comprising a micellar calcium phosphate-phosphopeptide complex and having a cariostatic function (Patent Document 7), a method that can accelerate remineralization and suppress caries by cleaning teeth using a dentifrice comprising a fluoride ion, and then allowing an oral liquid composition comprising a calcium ion to act (Patent Document 8), a dentifrice composition having a pH of 5 to 8 in which tricalcium phosphate is blended as a calcium salt powder having the ability to convert into hydroxyapatite when coming into contact with water in the oral cavity (Patent Document 9), and the like are proposed.

In addition, chewing gums and the like in which xylitol and a calcium phosphate, a noncrystalline calcium phosphate, or a phosphorylated oligosaccharide calcium are blended for accelerating remineralization are also produced, but remineralization is not always sufficient.

Lecithin is also referred to as phosphatidylcholine and is one lipid referred to as a phospholipid that is present in the cells of all animals and plants in the natural world. Lecithin is contained in the membranes of all cells in the body and is a main constituent of cell membranes that serve physiological functions. Lecithin is contained in a large amount in the brain, nervous tissue, and the like, is a component also needed when acetylcholine that is a neurotransmitter is made, is also associated with learning, memory, sleep, and the metabolism of lipids, and also has the function of protecting the liver. Lecithin includes "egg yolk lecithin" derived from egg yolk, and "soybean lecithin" derived from soybean. Egg yolk lecithin comprises a large amount of phosphatidylcholine and therefore is excellent in autonomic nerve and memory improvement and a preventing effect such as nerve disease prevention. Soybean lecithin has the characteristic of being able to remain long in a liquid and therefore is excellent in a preventing effect against arteriosclerosis, cerebral apoplexy, hyperlipemia, heart diseases, and the like. Examples of foodstuff comprising a large amount of lecithin include egg yolk, a soybean product, cereal, sesame oil, corn oil, a small fish, liver, and an eel. Health foods using lecithin extracted from these foods are sold. Lecithin is known to have emulsifying action, mold releasing action, oxidation preventing action, water retaining action, foaming and defoaming action, and the like and widely used in foods, industrial products, cosmetics, drugs, and the like as a natural emulsifier. In oral applications, using lecithin as an antioxidant (Patent Document 10), as an emulsifier (Patent Document 11), or as a surfactant (Patent Document 12 and Patent Document 13) is proposed. However, lecithin has no tooth remineralizing action.

## Prior Art Documents

### Patent Documents

Patent Document 1: Japanese unexamined Patent Application Publication No. 9-202717
Patent Document 2: Japanese unexamined Patent Application Publication No. 10-59814
Patent Document 3: Japanese unexamined Patent Application Publication No. 2000-128752
Patent Document 4: Japanese unexamined Patent Application Publication No. 2001-122748
Patent Document 5: Japanese unexamined Patent Application Publication No. 2005-314266
Patent Document 6: Japanese unexamined Patent Application Publication No. 2014-73989
Patent Document 7: Japanese unexamined Patent Application Publication No. 2006-213668
Patent Document 8: Japanese unexamined Patent Application Publication No. 2007-99632
Patent Document 9: Japanese unexamined Patent Application Publication No. 7-223930
Patent Document 10: Japanese unexamined Patent Application Publication No. 5-148125
Patent Document 11: Japanese unexamined Patent Application Publication No. 5-170632
Patent Document 12: Japanese unexamined Patent Application Publication (Translation of PCT Application) No. 2006-504776
Patent Document 13: Japanese unexamined Patent Application Publication No. 2013-129641

## Summary of the Invention

### Object to be Solved by the Invention

It is an object of the present invention to provide an oral composition having excellent effect of remineralizing demineralized tooth enamel.

### Means to Solve the Object

The present inventors have studied diligently in order to solve the above object, and as a result found that by blending, in combination, a calcium phosphate such as hydroxyapatite, calcium monohydrogen phosphate, or tricalcium phosphate, and lecithin conventionally known to have only emulsifying action, mold releasing action, oxidation preventing action, water retaining action, and foaming and defoaming action and having no tooth remineralizing action, and using the blend as an oral composition such as a dentifrice, the tooth remineralizing effect improves significantly, leading to the completion of the present invention.
Specifically, the present invention is specified by the items shown below.

(1) An oral composition comprising lecithin and a calcium phosphate, wherein a content of the calcium phosphate is 0.0001 to 30% by mass.
(2) The oral composition according to the above (1), wherein the lecithin is lecithin derived from soybean and/or lecithin derived from egg yolk.
(3) The oral composition according to the above (1) or (2), wherein the calcium phosphate is at least one calcium phosphate selected from the group consisting of hydroxyapatite, calcium monohydrogen phosphate, and tricalcium phosphate.
(4) The oral composition according to any one of the above (1) to (3), wherein a content of the lecithin is 0.001 to 10% by mass.
(5) The oral composition according to any one of the above (1) to (4), wherein the composition is a paste dentifrice, a powder dentifrice, a liquid dentifrice, or a mouthwash.
(6) A tooth remineralizing agent comprising lecithin and a calcium phosphate as active ingredients.

### Effect of the Invention

The oral composition of the present invention has excellent action of remineralizing demineralized tooth enamel by containing lecithin and a calcium phosphate such as hydroxyapatite, calcium monohydrogen phosphate, or tricalcium phosphate. In addition, the present invention can provide a tooth remineralizing agent having excellent remineralizing action that contains lecithin and a calcium phosphate such as hydroxyapatite, calcium monohydrogen phosphate, or tricalcium phosphate as active ingredients.

**Brief Description of Drawings**

[Figure 1] Figure 1 is a photograph showing the control surface and treated surface of a crown portion in Example 28 by a contact microradiogram (CMR).
[Figure 2] Figure 2 is a photograph showing the control surface and treated surface of a crown portion in Example 47 by a contact microradiogram (CMR).
[Figure 3] Figure 3 is a diagram in which the photograph shown in Figure 1 and Figure 2 is drawn using dark India ink, and the explanation of the state of each portion is added.

**Mode of Carrying Out the Invention**

The oral composition of the present invention is not particularly limited as long as it comprises lecithin and a calcium phosphate, and the content of the calcium phosphate is 0.0001 to 30% by mass. Specifically, a dentifrice such as a paste dentifrice, a powder dentifrice, and a liquid dentifrice, an oral cleaning agent such as a mouthwash and a gargling tablet, a troche, and the like can be illustrated. The tooth remineralizing agent of the present invention is not particularly limited as long as it contains lecithin and a calcium phosphate as active ingredients. Examples of the tooth remineralizing agent of the present invention can include tablet-like, powdery, pasty, liquid, and other remineralizing agents. The calcium phosphate in the present invention is not particularly limited as long as it is a calcium salt of phosphoric acid. Examples of the calcium phosphate in the present invention can include hydroxyapatite, calcium monohydrogen phosphate, and tricalcium phosphate. These calcium phosphates may each be used alone, two or more of these calcium phosphates may be used in combination. The calcium phosphate in the present invention may be a hydrate, or a calcium phosphate in which some of phosphorus and calcium are substituted by other elements such as magnesium, zinc, titanium, sodium, and potassium. When two or more calcium phosphates are used in combination, the total content of the calcium phosphates is 0.0001 to 30% by mass of the entire oral composition. As the calcium phosphate in the present invention, at least one calcium phosphate selected from the group consisting of hydroxyapatite, calcium monohydrogen phosphate, and tricalcium phosphate is preferred from the viewpoint of remineralization acceleration.
The content of the calcium phosphate in the present invention is preferably 0.01 to 30% by mass, more preferably 1.0 to 20% by mass, of the entire oral composition from the viewpoint of further improving the remineralizing effect and improving the feeling of use. The content of the calcium phosphate in the remineralizing agent of the present invention when the remineralizing agent is used in the oral cavity as it is preferably 0.0001 to 30% by mass, more preferably 0.01 to 30% by mass, further preferably 1.0 to 20% by mass, of the entire remineralizing agent from the viewpoint of further improving the remineralizing effect and improving the feeling of use. When the remineralizing agent is added to an oral composition or the like and used, the content of the calcium phosphate in the remineralizing agent of the present invention is preferably set so that the content of the calcium phosphate in the oral composition or the like after the remineralizing agent is added is in the above range. When two or more calcium phosphates are used in combination, the total content of the calcium phosphates is preferably in the above range. The oral composition and remineralizing agent of the present invention can significantly improve the remineralization of teeth because lecithin is blended together with a calcium phosphate such as hydroxyapatite, tricalcium phosphate, or calcium monohydrogen phosphate.
The hydroxyapatite that is one calcium phosphate in the present invention may be hydroxyapatite obtained as natural hard tissue from a fish bone of a food fish such as a salmon, a pig bone, a cow bone, or the like, in addition to hydroxyapatite synthesized by a usual method. Usually, hydroxyapatite is stoichiometrically represented by a composition consisting of $Ca_{10}(PO_4)_6(OH)_2$, but even nonstoichiometric hydroxyapatite not having a Ca/P molar ratio of 1.67 can have an apatite structure as well as exhibiting the properties of hydroxyapatite. For example, synthetic hydroxyapatite having a Ca/P molar ratio of about 1.4 to 1.8 is also included in the hydroxyapatite in the present invention.
The hydroxyapatite used in the present invention may be any of crystalline, low crystalline, and noncrystalline hydroxyapatite but is preferably low crystalline or noncrystalline hydroxyapatite (low crystalline hydroxyapatite and noncrystalline hydroxyapatite are hereinafter referred to as "amorphous hydroxyapatite") in terms of a caries preventing effect. "Low crystalline" refers to crystalline hydroxyapatite in which the X-ray diffraction peak is broader than that of a highly crystalline powder, and "noncrystalline" refers to hydroxyapatite in which the X-ray diffraction pattern shows a broad halo, and a diffraction pattern characteristic of a crystal is not obtained. Such amorphous hydroxyapatite can be obtained, for example, by freeze-drying apatite synthesized by a wet synthesis method or drying the apatite at a temperature of 100°C or less or firing the apatite at a temperature of about 300°C or less.
The content of hydroxyapatite in the oral composition of the present invention is 0.0001 to 30% by mass of the entire oral composition, and is preferably 0.01 to 30% by mass, more preferably 1.0 to 20% by mass, of the entire oral composition from the viewpoint of further improving the remineralizing effect and improving the feeling of use. Also in the case of the remineralizing agent of the present invention, similarly, the content of hydroxyapatite is preferably in the above range. When the remineralizing agent is added to an oral composition or the like and used, the content of hydroxyapatite in the remineralizing agent of the present invention is preferably set so that the content of hydroxyapatite in the oral composition or the like after the remineralizing agent is added is in the above range.

The calcium monohydrogen phosphate that is one calcium phosphate in the present invention is also referred to as calcium phosphate dibasic, is a compound represented by the chemical formula $CaHPO_4$ or $CaHPO_4 \cdot 2H_2O$, the dihydrate thereof, and is widely and generally used in drugs, foods, cosmetics, industrial raw materials, and the like. The calcium monohydrogen phosphate used in the present invention is not particularly limited as long as it can be used as a component of an oral composition. Examples of the calcium monohydrogen phosphate used in the present invention can include a product conforming to a standard such as the Japanese Standards of Food Additives, the Japanese Pharmacopoeia, or the Japanese Standards of Quasi-Drug Ingredients.

The tricalcium phosphate that is one calcium phosphate in the present invention is also referred to as tribasic calcium phosphate, is a compound represented by the chemical formula $Ca_3(PO_4)_2$, and is widely and generally used in foods, sundry goods, the petrochemical industry, and the like including drugs and cosmetics. The tricalcium phosphate used in the present invention is not particularly limited as long as it can be used as a component of an oral composition. Examples of the tricalcium phosphate used in the present invention can include a product conforming to a standard such as Japanese Pharmaceutical Excipients, the Japanese Standards of Quasi-Drug Ingredients, or Japanese Cosmetic Ingredients Codex.

The content of calcium monohydrogen phosphate and tricalcium phosphate in the present invention is 0.0001 to 30% by mass of the entire oral composition, and is preferably 0.01 to 30% by mass, more preferably 1.0 to 20% by mass, of the entire oral composition from the viewpoint of further improving remineralization and improving the feeling of use. Also in the case of the remineralizing agent of the present invention, similarly, the content of calcium monohydrogen phosphate and tricalcium phosphate is preferably in the above range. When the remineralizing agent is added to an oral composition or the like and used, the content of calcium monohydrogen phosphate and tricalcium phosphate in the remineralizing agent of the present invention is preferably set so that the content of calcium monohydrogen phosphate and tricalcium phosphate in the oral composition or the like after the remineralizing agent is added is in the above range.

In a case where a calcium phosphate is blended in an oral composition, when the content of the calcium phosphate is more than 40% by mass, the calcium phosphate is cakey due to water, and therefore the produced composition is hard, and the production may be difficult, the quality of the produced composition may be problematic, and the feeling of use may decrease. In the oral composition of the present invention, the content of the calcium phosphate is 0.0001 to 30% by mass of the entire oral composition, and therefore an oral composition without such problems can be obtained. In addition, in the oral composition of the present invention, by using lecithin and a calcium phosphate in combination, the remineralizing effect can be improved compared with when a calcium phosphate is used alone. Therefore, the calcium phosphate need not be excessively contained, and the content of the calcium phosphate can be decreased as needed, and therefore the flexibility of component blending design when the oral composition is prepared can be enhanced.

Lecithin is also referred to as phosphatidylcholine and is one of lipids referred to as phospholipids that are present in the cells of all animals and plants in the natural world. Lecithin is contained in the membranes of all cells in the body and is a main constituent of cell membranes that serve physiological functions. Examples of foodstuff comprising a large amount of lecithin include egg yolk, a soybean product, cereal, sesame oil, corn oil, a small fish, liver, and an eel. The lecithin in the present invention is not particularly limited. Examples of the lecithin in the present invention can include lecithin derived from egg yolk (egg yolk lecithin), lecithin derived from soybean (soybean lecithin), lecithin derived from rapeseed (rapeseed lecithin), lecithin derived from corn (corn lecithin), lecithin derived from a sunflower (sunflower lecithin), and lecithin derived from a peanut (peanut lecithin). These can each be used alone, or two or more of these can be used in combination. Enzyme-treated lecithins obtained by enzyme-treating these lecithins, enzymatically decomposed lecithins obtained by enzymatically decomposing these lecithins, and hydrogenated lecithins obtained by hydrogenation-treating these lecithins can also be used. Especially, egg yolk oil, enzymatically decomposed lecithin, and hydrogenated lecithin are preferred from the viewpoint of easy availability and the remineralization accelerating effect, and egg yolk oil, enzymatically decomposed or hydrogenated egg yolk lecithin, and enzymatically decomposed or hydrogenated soybean lecithin are more preferred. Specifically, as the egg yolk lecithin, a product such as Yoke Oil L-301 (Taiyo Kagaku Co., Ltd.), LPL-20S, PL-30S, LPL-20W, and PL-100P (Kewpie Corporation) can be illustrated, and as the soybean lecithin, a product such as Mensoft L-69, Sunlecithin A-1 (Taiyo Kagaku Co., Ltd.), BASIS LP-20, BASIS LP-20H, BASIS LS-60HR (The Nisshin OilliO Group, Ltd.), and SLP-White (Tsuji Oil Mills Co., Ltd.) can be illustrated. The lecithin in the present invention can be used in food and cosmetic applications without particular limitation.

The content of lecithin in the present invention is preferably 0.001 to 10% by mass, more preferably 0.01 to 5.0% by mass, more preferably 0.01 to 1.0% by mass, and further preferably 0.1 to 1.0% by mass of the entire oral composition from the viewpoint of the remineralization accelerating effect and the feeling of use.

The oral composition of the present invention can contain, in addition to the above-described components, additives such as an abrasive, a thickening agent, a binding agent, a humectant, a foaming agent, a flavoring, a sweetener, and a preservative, various active ingredients, and the like usually used in an oral composition. Specific examples of these components are shown below. In addition to these components shown below, an appropriate component according to the purpose, the type of composition, and the like can be further blended.

Examples of the abrasive can include calcium carbonate, calcium pyrophosphate, silica such as abrasive precipitated silica and abrasive gel silica, calcium silicate, aluminum silicate, aluminum oxide, aluminum hydroxide, alumina, zeolite, titanium oxide, zirconium silicate, insoluble sodium metaphosphate, magnesium tertiary phosphate, magnesium carbonate, calcium sulfate, magnesium sulfate, polymethyl methacrylate, bentonite, and a synthetic resin.

Examples of the thickening agent can include hydroxyethyl cellulose, carboxymethyl cellulose sodium, carrageenan, a carboxyvinyl polymer, xanthan gum, gelatin, pullulan, sodium alginate, sodium polyacrylate, polyvinyl alcohol, locust bean gum, guar gum, and hydroxypropylmethyl cellulose.

Examples of the binding agent can include methyl cellulose, propylene glycol alginate ester, pullulan, tragacanth gum, xanthan gum, pectin, furcellaran, chitosan, polyethylene oxide, polyvinylpyrrolidone, polyacrylic acid, polymethacrylic acid, peptone, casein, collagen, albumin, gum arabic, karaya gum, EUDRAGIT, ethyl cellulose, cellulose acetate, sodium polyacrylate, polyvinyl alcohol, polyvinyl acetal-dimethylaminoacetate, and cellulose acetate-dibutyl hydroxypropyl ether.

Examples of the emulsifier can include polyoxyethylene hydrogenated castor oil, sorbitan monostearate, a glycerin fatty acid ester, a propylene glycol fatty acid ester, an alkyl glyceryl ether, a polyoxyethylene sorbitol fatty acid ester, a polysorbate, polyoxyethylene, lauromacrogol, a sodium alkyl sulfate, an alkyl phosphate ester, a sodium alkylbenzene sulfonate, a sodium N-acyl sarcosinate, a N-acyl glutamate, a sucrose fatty acid ester, an alkyl glycoside, an alkyldimethylamine oxide, and an alkyl betaine.

Examples of the oil and fat components can include liquid paraffin, paraffin, a higher alcohol such as cetyl alcohol and stearyl alcohol, a fatty acid ester such as isopropyl myristate, lanolin, a fatty acid, an ester compound such as octyldodecyl myristate, diisopropyl adipate, hexadecyl isostearate, and decyl oleate, squalane, squalene, a medium chain fatty acid triglyceride, and a silicone.

Examples of the alcohol can include a lower alcohol such as ethanol, propyl alcohol, isopropyl alcohol, butanol, and isobutanol, and a polyhydric alcohol such as ethylene glycol, diethylene glycol, propylene glycol, dipropylene glycol, 1,3-butylene glycol, glycerin, 1,5-pentadiol, sorbitol, and polyethylene glycol.

As the surfactant, for example, as a nonionic surfactant, a sorbitan fatty acid ester, a glycerin fatty acid ester, a decaglycerin fatty acid ester, a polyglycerin fatty acid ester, decaglyceryl laurate, a propylene glycol-pentaerythritol fatty acid ester, a polyoxyethylene sorbitan fatty acid ester, a polyoxyethylene glycerin fatty acid ester, a polyoxyethylene sorbitol fatty acid ester, a polyethylene glycol fatty acid ester, a sucrose fatty acid ester, a polyoxyethylene alkyl ether, polyoxyethylene polyoxypropylene glycol, a polyoxypropylene alkyl ether, a polyoxyethylene polyoxypropylene alkyl ether, a polyoxyethylene alkyl phenyl ether, polyoxyethylene castor oil-hydrogenated castor oil, a polyoxyethylene lanolin-lanolin alcohol-beeswax derivative, a polyoxyethylene alkylamine-fatty acid amide, a polyoxyethylene alkyl phenyl formaldehyde condensate, a homogeneous polyoxyethylene alkyl ether, and the like can be illustrated. As an anionic surfactant, sodium lauryl sulfate, sodium myristyl sulfate, an alkyl sulfate, a polyoxyethylene alkyl sulfate, a N-acylamino acid and a salt thereof, a N-acylmethyltaurine and a salt thereof, a polyoxyethylene alkyl ether acetate, an alkyl sulfocarboxylate, an $\alpha$-olefin sulfonate, an alkyl phosphate, a polyoxyethylene alkyl ether phosphate, and the like can be illustrated. As a cationic surfactant, an alkylammonium, an alkylbenzylammonium salt, and the like can be illustrated. As an amphoteric surfactant, acetic acid betaine, imidazolinium betaine, and the like can be illustrated.

Examples of the pH adjusting agent can include citric acid and a salt thereof, phosphoric acid and a salt thereof, malic acid and a salt thereof, gluconic acid and a salt thereof, maleic acid and a salt thereof, aspartic acid and a salt thereof, gluconic acid and a salt thereof, succinic acid and a salt thereof, glucuronic acid and a salt thereof, fumaric acid and a salt thereof, glutamic acid and a salt thereof, adipic acid and a salt thereof, an inorganic acid such as hydrochloric acid, hydrofluoric acid, an alkali metal hydroxide such as sodium hydroxide and potassium hydroxide, and an amine such as triethanolamine, diethanolamine, and diisopropanolamine.

Examples of the stabilizer can include sodium sulfite, sodium pyrosulfite, sodium hydrogen sulfite, dibutylhydroxytoluene, butylhydroxyanisole, edetic acid or a salt thereof, vitamin C, vitamin E, and derivatives thereof.

Examples of the humectant can include a polyhydric alcohol such as glycerin, propylene glycol, polyethylene glycol, sorbitol, xylitol, ethylene glycol, 1,3-butylene glycol, and isopropylene glycol.

Examples of the foaming agent can include sodium lauryl sulfate, sodium N-lauroyl sarcosinate, and a nonionic surfactant.

Examples of the flavoring can include an essential oil such as menthol, peppermint, and spearmint, eucalyptus oil, orange oil, lemon oil, wintergreen oil, clove oil, peppermint oil, thyme oil, sage oil, carvone, linalool, eugenol, anethole, and herbal mint.

Examples of the sweetener can include saccharin sodium, aspartame, stevioside, neohesperidin dihydrochalcone, glycyrrhizin, aspartylphenylalanine methyl ester, acesulfame potassium, perillartine, p-methoxycinnamic aldehyde, and xylitol.

Examples of the preservative can include a p-hydroxybenzoate ester, an alkyldiaminoethylglycine hydrochloride, methylparaben, ethylparaben, and sodium benzoate.

Examples of other medicinal components can include allantoin, tocopherol acetate, isopropyl phenol, triclosan, chlorhexidine, chlorophyll, flavonoid, tranexamic acid, hinokitiol, cetylpyridinium chloride, sodium fluoride, stannous fluoride, sodium monofluorophosphate, dextranase, mutanase, protease, aminocaproic acid, glycyrrhizic acid, glycyrrhetic acid, azulene, allantoin, lysozyme chloride, a phellodendron bark extract, polyphosphoric acid, sodium chloride, an aloe squeezed juice, *Gynostemma pentaphyllum,* ginseng, an active oxygen removing agent, an antioxidant, an anti-inflammatory analgesic, an antihistamine, an antipruritic, a disinfectant, a vitamin preparation, and a hormone preparation.

For the amounts of these optional components blended, these optional components are appropriately used in ranges that do not hinder the effect of the present invention and are pharmaceutically allowable. The oral composition of the present invention can be produced by a usual method for producing an oral composition such as a paste dentifrice, a powder dentifrice, a liquid dentifrice, or a mouthwash. In the production of the oral composition of the present invention, lecithin, a calcium phosphate such as hydroxyapatite, calcium monohydrogen phosphate, tricalcium phosphate, and other components may be added in any process in the production process.

**Examples**

Paste dentifrices (Examples 1 to 101), liquid dentifrices (Examples 102 to 116), and mouthwashes (Examples 117 to 128), in which lecithin and hydroxyapatite, calcium monohydrogen phosphate, or tricalcium phosphate were blended were prepared and subjected to a remineralization test.

[Lecithin]

For the lecithin, as soybean lecithin, BASIS LP-20H (hydrogenated) (The Nisshin OilliO Group, Ltd.), SLP-White Lyso (enzymatically decomposed) (Tsuji Oil Mills Co., Ltd.), or lecithin (made of soybean, chemical) (NACALAI TESQUE, INC.) was used, and as egg yolk lecithin, Egg Yolk Lecithin PL-30S (egg yolk oil) (Kewpie Corporation) or Egg Yolk Lecithin PL-100P (hydrogenated) (Kewpie Corporation) was used.

[Hydroxyapatite]

A phosphoric acid aqueous solution having a concentration of 30% by mass was dropped into a calcium hydroxide suspension under stirring until a pH of 10 was reached, and the produced gel-like substance was allowed to stand at room temperature for 1 day for aging. Then, the gel-like substance was filtered by a glass filter, and the remaining substance was dried in air at 100°C to obtain a hydroxyapatite powder. The obtained hydroxyapatite powder had a maximum particle diameter of about 40 $\mu$m, a minimum particle diameter of about 0.05 $\mu$m, and an average particle diameter of about 5 $\mu$m. This hydroxyapatite powder was used.

[Calcium Monohydrogen Phosphate (Calcium Phosphate Dibasic)]

For the calcium monohydrogen phosphate, calcium hydrogen phosphate (food additive: YONEYAMA CHEMICAL INDUSTRY CO., LTD.) was used.

[Tricalcium Phosphate]

For the tricalcium phosphate, tricalcium phosphate (food additive: Taihei Chemical Industrial Co. Ltd.) was used.

As Comparative Examples, paste dentifrices, liquid dentifrices, mouthwashes, in which lecithin derived from soybean, lecithin derived from egg yolk, hydroxyapatite, calcium monohydrogen phosphate, or tricalcium phosphate were each blended were prepared with blends in Table 18 to Table 23, Table 27 to Table 30, Table 34, and Table 35 and subjected to a remineralization test.

1. Paste Dentifrices

[Table 1]

|  | Example 1 | Example 2 | Example 3 | Example 4 | Example 5 | Example 6 |
|---|---|---|---|---|---|---|
| Soybean lecithin (hydrogenated) | 10.0 | 0.5 | - | 0.001 | 5.0 | 0.5 |
| Egg yolk lecithin (egg yolk oil) | - | - | 0.5 | - | - | - |
| Hydroxyapatite | 30.0 | 30.0 | 30.0 | 30.0 | 20.0 | 20.0 |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 2]

|  | Example 7 | Example 8 | Example 9 | Example 10 | Example 11 | Example 12 |
|---|---|---|---|---|---|---|
| Soybean lecithin (hydrogenated) | 0.1 | 0.5 | 0.01 | 10.0 | - | - |
| Soybean lecithin (enzymatically decomposed) | - | - | - | - | 10.0 | - |
| Egg yolk lecithin (egg yolk oil) | - | - | - | - | - | 10.0 |
| Hydroxyapatite | 20.0 | 10.0 | 10.0 | 5.0 | 5.0 | 5.0 |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 3]

|  | Example 13 | Example 14 | Example 15 | Example 16 | Example 17 | Example 18 |
|---|---|---|---|---|---|---|
| Soybean lecithin (hydrogenated) | 1.0 | - | - | - | - | 0.5 |

(continued)

| | Example 13 | Example 14 | Example 15 | Example 16 | Example 17 | Example 18 |
|---|---|---|---|---|---|---|
| Soybean lecithin (enzymatically decomposed) | - | 1.0 | - | - | - | - |
| Soybean lecithin (chemical) | - | - | 1.0 | - | - | - |
| Egg yolk lecithin (egg yolk oil) | - | - | - | 1.0 | - | - |
| Egg yolk lecithin (hydrogenated) | - | - | - | - | 1.0 | - |
| Hydroxyapatite | 5.0 | 5.0 | 5.0 | 5.0 | 5.0 | 5.0 |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 4]

| | Example 19 | Example 20 | Example 21 | Example 22 | Example 23 | Example 24 |
|---|---|---|---|---|---|---|
| Soybean lecithin (hydrogenated) | - | - | - | - | 0.1 | - |
| Soybean lecithin (enzymatically decomposed) | 0.5 | - | - | - | - | 0.1 |
| Soybean lecithin (chemical) | - | 0.5 | - | - | - | - |
| Egg yolk lecithin (egg yolk oil) | - | - | 0.5 | - | - | - |
| Egg yolk lecithin (hydrogenated) | - | - | - | 0.5 | - | - |
| Hydroxyapatite | 5.0 | 5.0 | 5.0 | 5.0 | 5.0 | 5.0 |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |

(continued)

| | Example 19 | Example 20 | Example 21 | Example 22 | Example 23 | Example 24 |
|---|---|---|---|---|---|---|
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 5]

| | Example 25 | Example 26 | Example 27 | Example 28 | Example 29 | Example 30 |
|---|---|---|---|---|---|---|
| Soybean lecithin (hydrogenated) | - | - | - | 0.001 | - | - |
| Soybean lecithin (enzymatically decomposed) | - | - | - | - | 0.001 | - |
| Soybean lecithin (chemical) | 0.1 | - | - | - | - | - |
| Egg yolk lecithin (egg yolk oil) | - | 0.1 | - | - | - | 0.001 |
| Egg yolk lecithin (hydrogenated) | - | - | 0.1 | - | - | - |
| Hydroxyapatite | 5.0 | 5.0 | 5.0 | 5.0 | 5.0 | 5.0 |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 6]

| | Example 31 | Example 32 | Example 33 | Example 34 | Example 35 | Example 36 |
|---|---|---|---|---|---|---|
| Soybean lecithin (hydrogenated) | - | 5.0 | 0.5 | 1.0 | 0.01 | 5.0 |
| Egg yolk lecithin (hydrogenated) | 0.001 | - | - | | | |
| Hydroxyapatite | 5.0 | 1.0 | 1.0 | 0.1 | 0.1 | 0.01 |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |

(continued)

|  | Example 31 | Example 32 | Example 33 | Example 34 | Example 35 | Example 36 |
|---|---|---|---|---|---|---|
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 7]

|  | Example 37 | Example 38 | Example 39 | Example 40 | Example 41 |
|---|---|---|---|---|---|
| Soybean lecithin (hydrogenated) | 0.1 | 10.0 | 0.5 | - | 0.001 |
| Egg yolk lecithin (egg yolk oil) | - | - | - | 0.5 | - |
| Hydroxyapatite | 0.01 | 0.0001 | 0.0001 | 0.0001 | 0.0001 |
| Glycerin | 20.0 | 20.0 | 20.0 | 20.0 | 20.0 |
| Carboxymethyl cellulose | 2.0 | 2.0 | 2.0 | 2.0 | 2.0 |
| Silica | 5.0 | 5.0 | 5.0 | 5.0 | 5.0 |
| Polyethylene glycol | 3.0 | 3.0 | 3.0 | 3.0 | 3.0 |
| Sodium lauryl sulfate | 1.0 | 1.0 | 1.0 | 1.0 | 1.0 |
| Cetylpyridinium chloride | 0.1 | 0.1 | 0.1 | 0.1 | 0.1 |
| Menthol | 0.5 | 0.5 | 0.5 | 0.5 | 0.5 |
| Purified water | Balance | Balance | Balance | Balance | Balance |
| Total | 100.0 | 100.0 | 100.0 | 100.0 | 100.0 |

[Table 8]

|  | Example 42 | Example 43 | Example 44 | Example 45 | Example 46 | Example 47 |
|---|---|---|---|---|---|---|
| Soybean lecithin (hydrogenated) | 10.0 | 0.5 | 0.001 | 5.0 | 0.5 | 0.1 |
| Calcium monohydrogen phosphate | 30.0 | 30.0 | 30.0 | 20.0 | 20.0 | 20.0 |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 9]

| | Example 48 | Example 49 | Example 50 | Example 51 | Example 52 | Example 53 |
|---|---|---|---|---|---|---|
| Soybean lecithin (hydrogenated) | 0.5 | 0.01 | 10.0 | - | 1.0 | 0.5 |
| Soybean lecithin (enzymatically decomposed) | - | - | - | 10.0 | - | - |
| Calcium monohydrogen phosphate | 10.0 | 10.0 | 5.0 | 5.0 | 5.0 | 5.0 |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 10]

| | Example 54 | Example 55 | Example 56 | Example 57 | Example 58 | Example 59 |
|---|---|---|---|---|---|---|
| Soybean lecithin (hydrogenated) | - | - | - | - | 0.1 | - |
| Soybean lecithin (enzymatically decomposed) | 0.5 | - | - | - | - | 0.1 |
| Soybean lecithin (chemical) | - | 0.5 | - | - | - | - |
| Egg yolk lecithin (egg yolk oil) | - | - | 0.5 | - | - | - |
| Egg yolk lecithin (hydrogenated) | - | - | - | 0.5 | - | - |
| Calcium monohydrogen phosphate | 5.0 | 5.0 | 5.0 | 5.0 | 5.0 | 5.0 |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |

(continued)

|  | Example 54 | Example 55 | Example 56 | Example 57 | Example 58 | Example 59 |
|---|---|---|---|---|---|---|
| Purified water | Balance | Balance | Balance | Balance | Balance | Balance |
| Total | 100.0 | 100.0 | 100.0 | 100.0 | 100.0 | 100.0 |

[Table 11]

|  | Example 60 | Example 61 | Example 62 | Example 63 | Example 64 | Example 65 |
|---|---|---|---|---|---|---|
| Soybean lecithin (hydrogenated) | - | 0.001 | - | 5.0 | 0.5 | 1.0 |
| Soybean lecithin (chemical) | 0.1 | - | - | - | - | - |
| Egg yolk lecithin (egg yolk oil) | - | - | 0.001 | - | - | - |
| Calcium monohydrogen phosphate | 5.0 | 5.0 | 5.0 | 1.0 | 1.0 | 0.1 |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 12]

|  | Example 66 | Example 67 | Example 68 | Example 69 | Example 70 | Example 71 |
|---|---|---|---|---|---|---|
| Soybean lecithin (hydrogenated) | 0.01 | 5.0 | 0.1 | 10.0 | 0.5 | 0.001 |
| Calcium monohydrogen phosphate | 0.1 | 0.01 | 0.01 | 0.0001 | 0.0001 | 0.0001 |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |

(continued)

|  | Example 66 | Example 67 | Example 68 | Example 69 | Example 70 | Example 71 |
|---|---|---|---|---|---|---|
| Total | 100.0 | 100.0 | 100.0 | 100.0 | 100.0 | 100.0 |

[Table 13]

|  | Example 72 | Example 73 | Example 74 | Example 75 | Example 76 | Example 77 |
|---|---|---|---|---|---|---|
| Soybean lecithin (hydrogenated) | 10.0 | 0.5 | 0.001 | 5.0 | 0.5 | 0.1 |
| Tricalcium phosphate | 30.0 | 30.0 | 30.0 | 20.0 | 20.0 | 20.0 |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 14]

|  | Example 78 | Example 79 | Example 80 | Example 81 | Example 82 | Example 83 |
|---|---|---|---|---|---|---|
| Soybean lecithin (hydrogenated) | 0.5 | 0.01 | 10.0 | - | 1.0 | - |
| Soybean lecithin (enzymatically decomposed) | - | - | - | - | - | 1.0 |
| Egg yolk lecithin (egg yolk oil) | - | - | - | 10.0 | - | - |
| Tricalcium phosphate | 10.0 | 10.0 | 5.0 | 5.0 | 5.0 | 5.0 |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 15]

| | Example 84 | Example 85 | Example 86 | Example 87 | Example 88 | Example 89 |
|---|---|---|---|---|---|---|
| Soybean lecithin (hydrogenated) | - | 0.5 | - | - | - | - |
| Soybean lecithin (enzymatically decomposed) | - | - | 0.5 | - | - | - |
| Soybean lecithin (chemical) | 1.0 | - | - | 0.5 | - | - |
| Egg yolk lecithin (egg yolk oil) | - | - | - | - | 0.5 | - |
| Egg yolk lecithin (hydrogenated) | - | - | - | - | - | 0.5 |
| Tricalcium phosphate | 5.0 | 5.0 | 5.0 | 5.0 | 5.0 | 5.0 |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 16]

| | Example 90 | Example 91 | Example 92 | Example 93 | Example 94 | Example 95 |
|---|---|---|---|---|---|---|
| Soybean lecithin (hydrogenated) | 0.1 | 0.001 | - | 5.0 | 0.5 | 1.0 |
| Soybean lecithin (enzymatically decomposed) | - | - | 0.001 | - | - | - |
| Tricalcium phosphate | 5.0 | 5.0 | 5.0 | 1.0 | 1.0 | 0.1 |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 17]

| | Example 96 | Example 97 | Example 98 | Example 99 | Example 100 | Example 101 |
|---|---|---|---|---|---|---|
| Soybean lecithin (hydrogenated) | 0.01 | 5.0 | 0.1 | 10.0 | 0.5 | 0.001 |
| Tricalcium phosphate | 0.1 | 0.01 | 0.01 | 0.0001 | 0.0001 | 0.0001 |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 18]

| | Comparative Example 1 | Comparative Example 2 | Comparative Example 3 | Comparative Example 4 | Comparative Example 5 | Comparative Example 6 |
|---|---|---|---|---|---|---|
| Soybean lecithin (hydrogenated) | 0.001 | - | 0.01 | 0.1 | - | 0.5 |
| Egg yolk lecithin (egg yolk oil) | - | 0.001 | - | - | 0.1 | - |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 19]

| | Comparative Example 7 | Comparative Example 8 | Comparative Example 9 | Comparative Example 10 | Comparative Example 11 | Comparative Example 12 |
|---|---|---|---|---|---|---|
| Soybean lecithin (hydrogenated) | - | - | - | - | 1.0 | 10.0 |
| Soybean lecithin (enzymatically decomposed) | 0.5 | - | - | - | - | - |
| Soybean lecithin (chemical) | - | 0.5 | - | - | - | - |
| Egg yolk lecithin (egg yolk oil) | - | - | 0.5 | - | - | - |
| Egg yolk lecithin (hydrogenated) | - | - | - | 0.5 | - | - |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 20]

| | Comparative Example 13 | Comparative Example 14 | Comparative Example 15 | Comparative Example 16 | Comparative Example 17 | Comparative Example 18 |
|---|---|---|---|---|---|---|
| Hydroxyapatite | 0.0001 | 0.01 | 0.1 | 1.0 | 5.0 | 10.0 |
| Calcium monohydrogen phosphate | - | - | - | - | - | - |
| Tricalcium phosphate | - | - | - | - | - | - |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 21]

| | Comparative Example 19 | Comparative Example 20 | Comparative Example 21 | Comparative Example 22 | Comparative Example 23 | Comparative Example 24 |
|---|---|---|---|---|---|---|
| Hydroxyapatite | 20.0 | 30.0 | | | - | - | - |
| Calcium monohydrogen phosphate | - | - | 0.0001 | 0.01 | 0.1 | 1.0 |
| Tricalcium phosphate | - | - | - | - | - | - |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 22]

| | Comparative Example 25 | Comparative Example 26 | Comparative Example 27 | Comparative Example 28 | Comparative Example 29 | Comparative Example 30 |
|---|---|---|---|---|---|---|
| Hydroxyapatite | - | - | - | - | - | - |
| Calcium monohydrogen phosphate | 5.0 | 10.0 | 20.0 | 30.0 | - | - |
| Tricalcium phosphate | - | - | - | - | 0.0001 | 0.01 |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 23]

| | Comparative Example 31 | Comparative Example 32 | Comparative Example 33 | Comparative Example 34 | Comparative Example 35 | Comparative Example 36 |
|---|---|---|---|---|---|---|
| Hydroxyapatite | - | - | - | - | - | - |
| Calcium monohydrogen phosphate | - | - | - | - | - | - |
| Tricalcium phosphate | 0.1 | 1.0 | 5.0 | 10.0 | 20.0 | 30.0 |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

2. Liquid Dentifrices

[Table 24]

|  | Example 102 | Example 103 | Example 104 | Example 105 | Example 106 |
|---|---|---|---|---|---|
| Soybean lecithin (hydrogenated) | 0.001 | 0.5 | 0.01 | 0.1 | 1.0 |
| Hydroxyapatite | 10.0 | 5.0 | 1.0 | 0.1 | 0.001 |
| Xylitol | 10.0 | 10.0 | 10.0 | 10.0 | 10.0 |
| Carrageenan | 1.0 | 1.0 | 1.0 | 1.0 | 1.0 |
| Glycerin | 10.0 | 10.0 | 10.0 | 10.0 | 10.0 |
| Menthol | 0.4 | 0.4 | 0.4 | 0.4 | 0.4 |
| Purified water | Balance | Balance | Balance | Balance | Balance |
| Total | 100.0 | 100.0 | 100.0 | 100.0 | 100.0 |

[Table 25]

|  | Example 107 | Example 108 | Example 109 | Example 110 | Example 111 |
|---|---|---|---|---|---|
| Soybean lecithin (hydrogenated) | 0.001 | 0.5 | 0.01 | 0.1 | 1.0 |
| Calcium monohydrogen phosphate | 10.0 | 5.0 | 1.0 | 0.1 | 0.001 |
| Xylitol | 10.0 | 10.0 | 10.0 | 10.0 | 10.0 |
| Carrageenan | 1.0 | 1.0 | 1.0 | 1.0 | 1.0 |
| Glycerin | 10.0 | 10.0 | 10.0 | 10.0 | 10.0 |
| Menthol | 0.4 | 0.4 | 0.4 | 0.4 | 0.4 |
| Purified water | Balance | Balance | Balance | Balance | Balance |
| Total | 100.0 | 100.0 | 100.0 | 100.0 | 100.0 |

[Table 26]

|  | Example 112 | Example 113 | Example 114 | Example 115 | Example 116 |
|---|---|---|---|---|---|
| Soybean lecithin (hydrogenated) | 0.001 | 0.5 | 0.01 | 0.1 | 1.0 |
| Tricalcium phosphate | 10.0 | 5.0 | 1.0 | 0.1 | 0.001 |
| Xylitol | 10.0 | 10.0 | 10.0 | 10.0 | 10.0 |
| Carrageenan | 1.0 | 1.0 | 1.0 | 1.0 | 1.0 |
| Glycerin | 10.0 | 10.0 | 10.0 | 10.0 | 10.0 |
| Menthol | 0.4 | 0.4 | 0.4 | 0.4 | 0.4 |
| Purified water | Balance | Balance | Balance | Balance | Balance |
| Total | 100.0 | 100.0 | 100.0 | 100.0 | 100.0 |

[Table 27]

|  | Comparative Example 37 | Comparative Example 38 | Comparative Example 39 | Comparative Example 40 | Comparative Example 41 |
|---|---|---|---|---|---|
| Soybean lecithin (hydrogenated) | 0.001 | 0.01 | 0.1 | 0.5 | 1.0 |
| Xylitol | 10.0 | 10.0 | 10.0 | 10.0 | 10.0 |
| Carrageenan | 1.0 | 1.0 | 1.0 | 1.0 | 1.0 |
| Glycerin | 10.0 | 10.0 | 10.0 | 10.0 | 10.0 |
| Menthol | 0.4 | 0.4 | 0.4 | 0.4 | 0.4 |
| Purified water | Balance | Balance | Balance | Balance | Balance |
| Total | 100.0 | 100.0 | 100.0 | 100.0 | 100.0 |

[Table 28]

|  | Comparative Example 42 | Comparative Example 43 | Comparative Example 44 | Comparative Example 45 | Comparative Example 46 |
|---|---|---|---|---|---|
| Hydroxyapatite | 10.0 | 5.0 | 1.0 | 0.1 | 0.001 |
| Xylitol | 10.0 | 10.0 | 10.0 | 10.0 | 10.0 |
| Carrageenan | 1.0 | 1.0 | 1.0 | 1.0 | 1.0 |
| Glycerin | 10.0 | 10.0 | 10.0 | 10.0 | 10.0 |
| Menthol | 0.4 | 0.4 | 0.4 | 0.4 | 0.4 |
| Purified water | Balance | Balance | Balance | Balance | Balance |
| Total | 100.0 | 100.0 | 100.0 | 100.0 | 100.0 |

[Table 29]

|  | Comparative Example 47 | Comparative Example 48 | Comparative Example 49 | Comparative Example 50 | Comparative Example 51 |
|---|---|---|---|---|---|
| Calcium monohydrogen phosphate | 10.0 | 5.0 | 1.0 | 0.1 | 0.001 |
| Xylitol | 10.0 | 10.0 | 10.0 | 10.0 | 10.0 |
| Carrageenan | 1.0 | 1.0 | 1.0 | 1.0 | 1.0 |
| Glycerin | 10.0 | 10.0 | 10.0 | 10.0 | 10.0 |
| Menthol | 0.4 | 0.4 | 0.4 | 0.4 | 0.4 |
| Purified water | Balance | Balance | Balance | Balance | Balance |
| Total | 100.0 | 100.0 | 100.0 | 100.0 | 100.0 |

[Table 30]

|  | Comparative Example 52 | Comparative Example 53 | Comparative Example 54 | Comparative Example 55 | Comparative Example 56 |
|---|---|---|---|---|---|
| Tricalcium phosphate | 10.0 | 5.0 | 1.0 | 0.1 | 0.001 |

(continued)

|  | Comparative Example 52 | Comparative Example 53 | Comparative Example 54 | Comparative Example 55 | Comparative Example 56 |
|---|---|---|---|---|---|
| Xylitol | 10.0 | 10.0 | 10.0 | 10.0 | 10.0 |
| Carrageenan | 1.0 | 1.0 | 1.0 | 1.0 | 1.0 |
| Glycerin | 10.0 | 10.0 | 10.0 | 10.0 | 10.0 |
| Menthol | 0.4 | 0.4 | 0.4 | 0.4 | 0.4 |
| Purified water | Balance | Balance | Balance | Balance | Balance |
| Total | 100.0 | 100.0 | 100.0 | 100.0 | 100.0 |

3. Mouthwashes

[Table 31]

|  | Example 117 | Example 118 | Example 119 | Example 120 |
|---|---|---|---|---|
| Soybean lecithin (hydrogenated) | 0.001 | 0.1 | 0.01 | 0.5 |
| Hydroxyapatite | 0.1 | 0.01 | 0.001 | 0.0001 |
| Ethyl alcohol | 10.0 | 10.0 | 10.0 | 10.0 |
| Sodium lauryl sulfate | 1.0 | 1.0 | 1.0 | 1.0 |
| Glycerin | 10.0 | 10.0 | 10.0 | 10.0 |
| Menthol | 0.4 | 0.4 | 0.4 | 0.4 |
| Purified water | Balance | Balance | Balance | Balance |
| Total | 100.0 | 100.0 | 100.0 | 100.0 |

[Table 32]

|  | Example 121 | Example 122 | Example 123 | Example 124 |
|---|---|---|---|---|
| Soybean lecithin (hydrogenated) | 0.001 | 0.1 | 0.01 | 0.5 |
| Calcium monohydrogen phosphate | 0.1 | 0.01 | 0.001 | 0.0001 |
| Ethyl alcohol | 10.0 | 10.0 | 10.0 | 10.0 |
| Sodium lauryl sulfate | 1.0 | 1.0 | 1.0 | 1.0 |
| Glycerin | 10.0 | 10.0 | 10.0 | 10.0 |
| Menthol | 0.4 | 0.4 | 0.4 | 0.4 |
| Purified water | Balance | Balance | Balance | Balance |
| Total | 100.0 | 100.0 | 100.0 | 100.0 |

[Table 33]

|  | Example 125 | Example 126 | Example 127 | Example 128 |
|---|---|---|---|---|
| Soybean lecithin (hydrogenated) | 0.001 | 0.1 | 0.01 | 0.5 |
| Tricalcium phosphate | 0.1 | 0.01 | 0.001 | 0.0001 |
| Ethyl alcohol | 10.0 | 10.0 | 10.0 | 10.0 |

(continued)

| | Example 125 | Example 126 | Example 127 | Example 128 |
|---|---|---|---|---|
| Sodium lauryl sulfate | 1.0 | 1.0 | 1.0 | 1.0 |
| Glycerin | 10.0 | 10.0 | 10.0 | 10.0 |
| Menthol | 0.4 | 0.4 | 0.4 | 0.4 |
| Purified water | Balance | Balance | Balance | Balance |
| Total | 100.0 | 100.0 | 100.0 | 100.0 |

[Table 34]

| | Comparative Example 57 | Comparative Example 58 |
|---|---|---|
| Soybean lecithin (hydrogenated) | 0.001 | 0.5 |
| Ethyl alcohol | 10.0 | 10.0 |
| Sodium lauryl sulfate | 1.0 | 1.0 |
| Glycerin | 10.0 | 10.0 |
| Menthol | 0.4 | 0.4 |
| Purified water | Balance | Balance |
| Total | 100.0 | 100.0 |

[Table 35]

| | Comparative Example 59 | Comparative Example 60 | Comparative Example 61 | Comparative Example 62 | Comparative Example 63 | Comparative Example 64 |
|---|---|---|---|---|---|---|
| Hydroxyapatite | 0.0001 | 0.1 | - | - | - | - |
| Calcium monohydrogen phosphate | - | - | 0.0001 | 0.1 | - | - |
| Tricalcium phosphate | - | - | - | - | 0.0001 | 0.1 |
| Ethyl alcohol | 10.0 | 10.0 | 10.0 | 10.0 | 10.0 | 10.0 |
| Sodium lauryl sulfate | 1.0 | 1.0 | 1.0 | 1.0 | 1.0 | 1.0 |
| Glycerin | 10.0 | 10.0 | 10.0 | 10.0 | 10.0 | 10.0 |
| Menthol | 0.4 | 0.4 | 0.4 | 0.4 | 0.4 | 0.4 |
| Purified water | Balance | Balance | Balance | Balance | Balance | Balance |
| Total | 100.0 | 100.0 | 100.0 | 100.0 | 100.0 | 100.0 |

[Remineralization Test Method]

In order to confirm the remineralizing effect, a previously made artificial incipient caries test sample was used. For the making of the artificial incipient caries test sample, enamel on the labial surface of the crown portion of a bovine front tooth was used. The enamel surface was abraded with #1000, #2400, and #4000 abrasive paper. A window of about 5 × 3 mm was made with Nail Enamel (Manufactured by Shiseido Company, Limited) at a site to be tested, on the abraded enamel surface, and immersed in a 0.1 M lactate buffer solution (pH 4.8, 3.0 mmM CaCl$_2$, 1.8 mmM KH$_2$PO$_4$, 1.0% CMC) at 37°C for 4 days to make artificial incipient caries. For the control for the test, the crown top side half of the window of about 5 × 3 mm was further masked with Nail Enamel (Manufactured by Shiseido Company, Limited) to provide a site to be compared (control). A paste dentifrice, a liquid dentifrice, or a mouthwash was mixed with purified water to form a suspension solution, and the suspension solution was used as a test solution (test substance).

In the remineralization test, the artificial incipient caries specimen made above was immersed in each test solution for 12 days, then the specimen was cut parallel to the tooth axis to a thickness of about 500 μm by a microcutter, and then this section was abraded under water pouring using #1000, #2400, and #4000 abrasive paper so as to provide a parallel thin section having a thickness of about 100 μm.

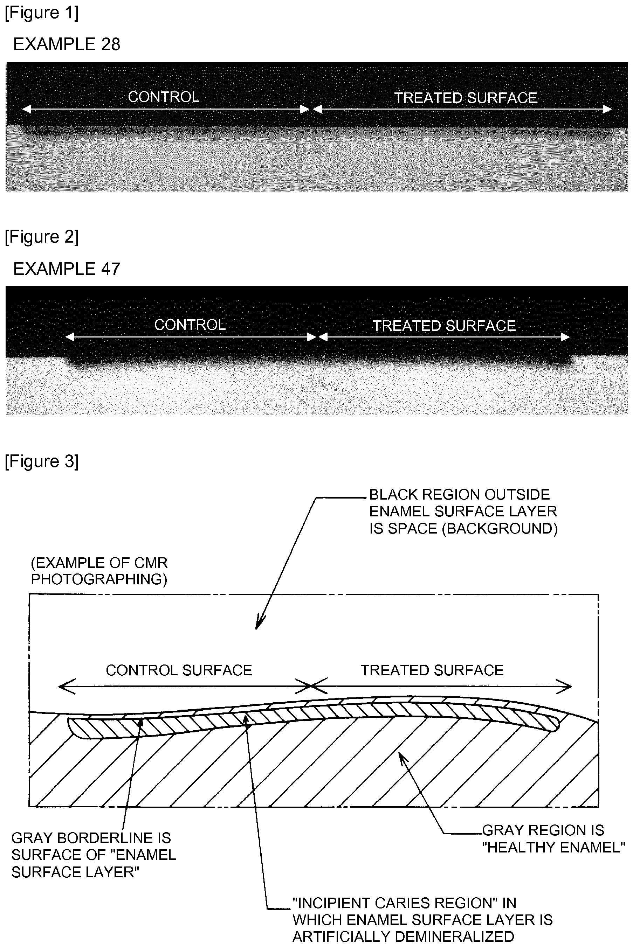

After the abrading, contact microradiogram (CMR) photographing was performed (see Figure 1 and Figure 2) in order to check the tooth remineralizing effect. The "CONTROL SURFACE" in the figure is a portion to be compared for to what extent the oral composition of an Examples and Comparative Examples has the remineralizing effect, and the state of the artificial incipient caries is maintained. The "CONTROL SURFACE" is a half portion of the artificial incipient caries (window of about 5 × 3 mm) region. The "TREATED SURFACE" in the figure is a portion in which the test solution (test substance) of the Examples and Comparative Examples is allowed to act.

The effect of remineralizing the artificial incipient caries site was analyzed using a computer. In image analysis by the computer, the amount of the remineralized mineral was calculated based on the formula of Angmer et al. (B. Angmer, D. Carlstrom and J. E. Glas: Studies on Ultrastructure of Dental Enemel IV: The Mineralization of normal Human Enamel, J. Ultrastructure. Res. 8, 12-23, 1963), and the amounts of the mineral lost ΔZ (% volume mineral•μm) at the control surface and treated surface of each section were calculated according to the method of Damato et al. (F. A. Damato, R. Stang and K. W. Stephen: Effect of Fluoride Concentration on Reminerelization of Carious Enamel: an in vitro pH-Cycling Study, Caries Res, 24, 174-180, 1990). The remineralization rate was calculated by the following formula:

[Equation 1]

$$\text{remineralization rate} = \frac{\Delta Z \text{ at control surface} - \Delta Z \text{ at treated surface}}{\Delta Z \text{ at control surface}} \times 100 \, (\%)$$

Table 31 to Table 36 show the results of confirming the remineralizing effect of the oral compositions by such a computer image analysis method.

1. Paste Dentifrices

[Table 36]

| Example | Example 1 | Example 2 | Example 3 | Example 4 | Example 5 | Example 6 |
|---|---|---|---|---|---|---|
| Remineralization rate (%) | 22.0 | 41.5 | 41.6 | 19.5 | 25.2 | 41.3 |
| Example | Example 7 | Example 8 | Example 9 | Example 10 | Example 11 | Example 12 |
| Remineralization rate (%) | 37.6 | 39.9 | 24.6 | 19.0 | 18.9 | 19.2 |
| Example | Example 13 | Example 14 | Example 15 | Example 16 | Example 17 | Example 18 |
| Remineralization rate (%) | 34.7 | 34.6 | 35.1 | 34.4 | 34.3 | 39.2 |
| Example | Example 19 | Example 20 | Example 21 | Example 22 | Example 23 | Example 24 |
| Remineralization rate (%) | 39.4 | 40.3 | 39.8 | 40.5 | 35.3 | 35.3 |

(continued)

| Example | Example 25 | Example 26 | Example 27 | Example 28 | Example 29 | Example 30 |
|---|---|---|---|---|---|---|
| Remineralization rate (%) | 36.0 | 35.9 | 35.9 | 16.9 | 16.8 | 16.7 |
| Example | Example 31 | Example 32 | Example 33 | Example 34 | Example 35 | Example 36 |
| Remineralization rate (%) | 16.6 | 21.9 | 38.4 | 32.0 | 20.8 | 17.0 |
| Example | Example 37 | Example 38 | Example 39 | Example 40 | Example 41 | |
| Remineralization rate (%) | 29.3 | 10.8 | 31.1 | 30.9 | 9.1 | |
| Example | Example 42 | Example 43 | Example 44 | Example 45 | Example 46 | Example 47 |
| Remineralization rate (%) | 10.5 | 27.9 | 8.5 | 12.8 | 27.8 | 25.8 |
| Example | Example 48 | Example 49 | Example 50 | Example 51 | Example 52 | Example 53 |
| Remineralization rate (%) | 27.5 | 12.3 | 9.9 | 9.6 | 24.1 | 27.3 |
| Example | Example 54 | Example 55 | Example 56 | Example 57 | Example 58 | Example 59 |
| Remineralization rate (%) | 27.4 | 27.4 | 27.0 | 27.2 | 25.2 | 25.1 |
| Example | Example 60 | Example 61 | Example 62 | Example 63 | Example 64 | Example 65 |
| Remineralization rate (%) | 25.5 | 8.0 | 7.9 | 12.4 | 27.2 | 23.5 |
| Example | Example 66 | Example 67 | Example 68 | Example 69 | Example 70 | Example 71 |
| Remineralization rate (%) | 11.6 | 12.0 | 24.6 | 8.7 | 26.4 | 7.3 |
| Example | Example 72 | Example 73 | Example 74 | Example 75 | Example 76 | Example 77 |
| Remineralization rate (%) | 10.1 | 17.1 | 10.7 | 13.1 | 17.1 | 16.4 |
| Example | Example 78 | Example 79 | Example 80 | Example 81 | Example 82 | Example 83 |
| Remineralization rate (%) | 16.6 | 12.8 | 8.9 | 8.3 | 15.5 | 15.6 |
| Example | Example 84 | Example 85 | Example 86 | Example 87 | Example 88 | Example 89 |
| Remineralization rate (%) | 16.0 | 15.9 | 15.8 | 16.6 | 15.7 | 15.9 |
| Example | Example 90 | Example 91 | Example 92 | Example 93 | Example 94 | Example 95 |
| Remineralization rate (%) | 15.1 | 9.2 | 9.0 | 11.1 | 15.2 | 13.0 |
| Example | Example 96 | Example 97 | Example 98 | Example 99 | Example 100 | Example 101 |
| Remineralization rate (%) | 9.5 | 8.9 | 11.9 | 4.3 | 11.7 | 3.9 |

[Table 37]

| Example | Comparative Example 1 | Comparative Example 2 | Comparative Example 3 | Comparative Example 4 | Comparative Example 5 | Comparative Example 6 |
|---|---|---|---|---|---|---|
| Remineralization rate (%) | -9.0 | -13.6 | -15.9 | -14.2 | -16.1 | -8.6 |
| Example | Comparative Example 7 | Comparative Example 8 | Comparative Example 9 | Comparative Example 10 | Comparative Example 11 | Comparative Example 12 |
| Remineralization rate (%) | -8.4 | -11.0 | -13.5 | -8.1 | -9.7 | -16.3 |
| Example | Comparative Example 13 | Comparative Example 14 | Comparative Example 15 | Comparative Example 16 | Comparative Example 17 | Comparative Example 18 |
| Remineralization rate (%) | 2.3 | 4.6 | 7.8 | 9.6 | 10.4 | 11.3 |
| Example | Comparative Example 19 | Comparative Example 20 | Comparative Example 21 | Comparative Example 22 | Comparative Example 23 | Comparative Example 24 |
| Remineralization rate (%) | 11.5 | 13.2 | 0.9 | 1.5 | 1.6 | 1.9 |
| Example | Comparative Example 25 | Comparative Example 26 | Comparative Example 27 | Comparative Example 28 | Comparative Example 29 | Comparative Example 30 |
| Remineralization rate (%) | 2.0 | 2.1 | 2.4 | 2.7 | 1.4 | 2.6 |
| Example | Comparative Example 31 | Comparative Example 32 | Comparative Example 33 | Comparative Example 34 | Comparative Example 35 | Comparative Example 36 |
| Remineralization rate (%) | 3.2 | 5.0 | 5.8 | 6.5 | 7.1 | 7.2 |

2. Liquid Dentifrices

[Table 38]

| Example | Example 102 | Example 103 | Example 104 | Example 105 | Example 106 |
|---|---|---|---|---|---|
| Remineralization rate (%) | 17.7 | 39.4 | 22.2 | 32.6 | 26.7 |
| Example | Example 107 | Example 108 | Example 109 | Example 110 | Example 111 |
| Remineralization rate (%) | 8.2 | 27.2 | 11.9 | 24.6 | 23.1 |
| Example | Example 112 | Example 113 | Example 114 | Example 115 | Example 116 |
| Remineralization rate (%) | 10.0 | 15.4 | 11.1 | 12.7 | 11.6 |

[Table 39]

| Example | Comparative Example 37 | Comparative Example 38 | Comparative Example 39 | Comparative Example 40 | Comparative Example 41 |
|---|---|---|---|---|---|
| Remineralization rate (%) | -8.7 | -8.1 | -9.5 | -16.8 | -16.2 |
| Example | Comparative Example 42 | Comparative Example 43 | Comparative Example 44 | Comparative Example 45 | Comparative Example 46 |
| Remineralization rate (%) | 10.7 | 9.8 | 9.0 | 7.1 | 2.4 |
| Example | Comparative Example 47 | Comparative Example 48 | Comparative Example 49 | Comparative Example 50 | Comparative Example 51 |
| Remineralization rate (%) | 2.3 | 2.1 | 1.8 | 1.5 | 1.5 |
| Example | Comparative Example 52 | Comparative Example 53 | Comparative Example 54 | Comparative Example 55 | Comparative Example 56 |
| Remineralization rate (%) | 5.9 | 5.6 | 4.9 | 3.9 | 2.5 |

3. Mouthwashes

[Table 40]

| Example | Example 117 | Example 118 | Example 119 | Example 120 |
|---|---|---|---|---|
| Remineralization rate (%) | 14.8 | 28.9 | 14.9 | 31.8 |
| Example | Example 121 | Example 122 | Example 123 | Example 124 |
| Remineralization rate (%) | 7.5 | 24.5 | 11.2 | 26.6 |
| Example | Example 125 | Example 126 | Example 127 | Example 128 |
| Remineralization rate (%) | 6.9 | 12.0 | 8.9 | 12.3 |

[Table 41]

| Example | Comparative Example 57 | Comparative Example 58 | | | | |
|---|---|---|---|---|---|---|
| Remineralization rate (%) | -10.1 | -8.6 | | | | |
| Example | Comparative Example 59 | Comparative Example 60 | Comparative Example 61 | Comparative Example 62 | Comparative Example 63 | Comparative Example 64 |
| Remineralization rate (%) | 2.7 | 8.6 | 1.3 | 1.8 | 1.6 | 3.7 |

As shown in Comparative Examples 1 to 12, Comparative Examples 37 to 41, and Comparative Examples 57 and 58, for both soybean lecithin and egg yolk lecithin, with lecithin alone, a tooth demineralization tendency is seen to some extent, rather than having no remineralizing effect.

In contrast to this, in the Examples in which lecithin was blended together with hydroxyapatite, calcium monohydrogen phosphate, or tricalcium phosphate, in all cases, the large improving effect of the tooth remineralizing effect was seen compared with Comparative Examples 13 to 36, Comparative Examples 42 to 56, and Comparative Examples 59 to 64 in which hydroxyapatite, calcium monohydrogen phosphate, or tricalcium phosphate was used alone.

No difference was noted in the remineralizing effect when various soybean lecithins and egg yolk lecithins were blended together with the above various calcium phosphates (Examples 2 and 3, Examples 10 to 12, Examples 13 to 17, Examples 18 to 22, Examples 23 to 27, Examples 28 to 31, Examples 39 and 40, Examples 53 to 57, Examples 61 and 62, Examples 80 and 81, Examples 82 to 84, Examples 85 to 89, and Examples 91 and 92). Therefore, it is found that whether derived from soybean or egg yolk, lecithin can be similarly used in applications for improving the remineralization of teeth.

**Industrial Applicability**

The oral composition of the present invention is very excellent in the remineralization of teeth, can be used for various dentifrices such as paste dentifrices, powder dentifrices, liquid dentifrices, and mouthwashes, and other applications for improving the remineralization of teeth in the oral cavity, and has high industrial usefulness.

**Claims**

1. An oral composition comprising lecithin and a calcium phosphate, wherein a content of the calcium phosphate is 0.0001 to 30% by mass.

2. The oral composition according to claim 1, wherein the lecithin is lecithin derived from soybean and/or lecithin derived from egg yolk.

3. The oral composition according to claim 1 or 2, wherein the calcium phosphate is at least one calcium phosphate selected from the group consisting of hydroxyapatite, calcium monohydrogen phosphate, and tricalcium phosphate.

4. The oral composition according to any one of claims 1 to 3, wherein a content of the lecithin is 0.001 to 10% by mass.

5. The oral composition according to any one of claims 1 to 4, wherein the composition is a paste dentifrice, a powder dentifrice, a liquid dentifrice, or a mouthwash.

6. A tooth remineralizing agent comprising lecithin and a calcium phosphate as active ingredients.

[Figure 1]

EXAMPLE 28

CONTROL · TREATED SURFACE

[Figure 2]

EXAMPLE 47

CONTROL · TREATED SURFACE

[Figure 3]

BLACK REGION OUTSIDE ENAMEL SURFACE LAYER IS SPACE (BACKGROUND)

(EXAMPLE OF CMR PHOTOGRAPHING)

CONTROL SURFACE · TREATED SURFACE

GRAY BORDERLINE IS SURFACE OF "ENAMEL SURFACE LAYER"

GRAY REGION IS "HEALTHY ENAMEL"

"INCIPIENT CARIES REGION" IN WHICH ENAMEL SURFACE LAYER IS ARTIFICIALLY DEMINERALIZED

**INTERNATIONAL SEARCH REPORT**

| International application No. |
|---|
| PCT/JP2016/004885 |

| A. CLASSIFICATION OF SUBJECT MATTER |
|---|
| *A61K8/55*(2006.01)i, *A61K8/24*(2006.01)i, *A61Q11/00*(2006.01)i |

According to International Patent Classification (IPC) or to both national classification and IPC

| B. FIELDS SEARCHED |
|---|

Minimum documentation searched (classification system followed by classification symbols)
A61K8/55, A61K8/24, A61Q11/00, A61K6/00, A23G3/00, A23G4/00, A23L33/00

| Documentation searched other than minimum documentation to the extent that such documents are included in the fields searched |
|---|
| Jitsuyo Shinan Koho          1922-1996   Jitsuyo Shinan Toroku Koho   1996-2017 |
| Kokai Jitsuyo Shinan Koho    1971-2017   Toroku Jitsuyo Shinan Koho   1994-2017 |

Electronic data base consulted during the international search (name of data base and, where practicable, search terms used)
JSTPlus/JMEDPlus/JST7580(JDreamIII)

| C. DOCUMENTS CONSIDERED TO BE RELEVANT |
|---|

| Category* | Citation of document, with indication, where appropriate, of the relevant passages | Relevant to claim No. |
|---|---|---|
| X | WO 2014/160285 A1 (OKAY, Devin, J), 02 October 2014 (02.10.2014), claims 7 to 9, 32; page 19, lines 8 to 18; examples 1, 2 & JP 2016-519064 A        & US 2015/0231060 A1 & US 2015/0290107 A1    & EP 2968084 A1 & CA 2905991 A1          & CN 105142596 A | 1-6 |
| X | JP 2005-314250 A (Ikko Chemical Co., Ltd.), 10 November 2005 (10.11.2005), paragraphs [0011], [0014]; example 1 (Family: none) | 1-6 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 27 January 2017 (27.01.17) | 07 February 2017 (07.02.17) |

| Name and mailing address of the ISA/ | Authorized officer |
|---|---|
| Japan Patent Office 3-4-3,Kasumigaseki,Chiyoda-ku, Tokyo 100-8915,Japan | Telephone No. |

Form PCT/ISA/210 (second sheet) (January 2015)

**INTERNATIONAL SEARCH REPORT**

| International application No. |
|---|
| PCT/JP2016/004885 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2014-110818 A  (Intercontinental Great Brands LLC), 19 June 2014 (19.06.2014), entire text & JP 2009-532065 A        & JP 2009-532478 A & JP 2011-200256 A        & US 2007/0237805 A1 entire text & WO 2007/117536 A2      & WO 2007/117537 A3 & EP 2001452 B1          & EP 2026745 A4 & CN 101415393 A        & CN 101415406 A | 1-6 |
| A | JP 64-047712 A  (Taiyo Kagaku Co., Ltd.), 22 February 1989 (22.02.1989), example 1 (Family: none) | 1-6 |
| A | JP 05-170632 A  (Block Drag Co., Inc.), 09 July 1993 (09.07.1993), examples 4 to 6 & US 5120528 A examples 4 to 6 & EP 518608 A1              & DE 69201677 T2 & CA 2071146 A | 1-6 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9202717 A
- JP 10059814 A
- JP 2000128752 A
- JP 2001122748 A
- JP 2005314266 A
- JP 2014073989 A
- JP 2006213668 A
- JP 2007099632 A
- JP 7223930 A
- JP 5148125 A
- JP 5170632 A
- JP 2006504776 PCT
- JP 2013129641 A

**Non-patent literature cited in the description**

- **B. ANGMER ; D. CARLSTROM ; J. E. GLAS.** Studies on Ultrastructure of Dental Enemel IV: The Mineralization of normal Human Enamel. *J. Ultrastructure. Res.,* 1963, vol. 8, 12-23
- **F. A. DAMATO ; R. STANG ; K. W. STEPHEN.** Effect of Fluoride Concentration on Reminerelization of Carious Enamel: an in vitro pH-Cycling Study. *Caries Res,* 1990, vol. 24, 174-180